Europäisches Patentamt

European Patent Office

Office européen des brevets

(11) Numéro de publication: **0 236 853**

**A1**

(12) **DEMANDE DE BREVET EUROPEEN**

(21) Numéro de dépôt: 87.102672.0

(22) Date de dépôt: 25.02.87

(51) Int. Cl.⁴: **A61M 1/00 , A61M 27/00**

(30) Priorité: 06.03.86 FR 8603321

(43) Date de publication de la demande:
16.09.87 Bulletin 87/38

(84) Etats contractants désignés:
CH DE GB LI

(71) Demandeur: IMTEC S.A.
Postfach 701
CH-8025 Zürich(CH)

(72) Inventeur: deCouet, Alexandre
Weinbergstrasse 115
CH-8006 Zürich(CH)
Inventeur: Hamid, Mohamed
18, rue du Canal de la Marne
F-67300 Schiltigheim(FR)

(74) Mandataire: Caron, Gérard
ICB Ingénieurs Conseils en Brevets SA
Faubourg du Lac 6
CH-2501 Bienne(CH)

(54) **Drain chirurgical.**

(57) Ce drain comporte un tube (1) pourvu d'orifices - (4) d'aspiration des sécrétions résiduelles receuillies à un site opératoire. Dans ce tube est délimité un volume (9) de capacité variable. Ce volume est réparti en une ou plusieurs poches gonflables (9-1, 9-2..9-n) délimitées entre la paroi intérieure du tube - (1) et une gaine (8) fixée judicieusement contre cette paroi. Cette gaine est en une matière élastique. Une source à dépression (6, 7) est raccordée au canal laissé libre dans le tube par la gaine (8). Par ailleurs, une source de fluide (18) peut être raccordée au volume (9) délimité par cette gaine pour en permettre le changement de capacité. Ce changement a pour effet de décolmater les orifices d'aspiration (4).

Application aux drains chirurgicaux utilisés pour évacuer en phase postopératoire toutes sécrétions apparaissant dans le site de l'opération.

Fig. 1

## DRAIN CHIRURGICAL

La présente invention concerne les drains chirurgicaux utilisés principalement pour évacuer en phase postopératoire du site chirurgical toute substance intervenant de façon défavorable à cet endroit.

Plus particulièrement la fonction idéale et fondamentale de tout drainage consiste à évacuer au dehors de la cavité abdominale, thoracique ou pelvienne:

a) toute collection susceptible de se former à la suite d'une intervention chirurgicale;

b) toute hémorragie importante ou distillée afin que le chirurgien puisse réintervenir rapidement, car le drain laissé dans le site chirurgical constitue une soupape sentinelle de toute complication;

c) toute fuite anormale de substance organique dont l'évacuation épargne les organes avoisinant le site chirurgical, notamment si cette substance est corrosive ou infectante.

Or, actuellement, la majorité des drains chirurgicaux connus ont tendance à s'obstruer dans un délai variant d'entre quelques minutes et quelques heures après leur installation, suivant les régions anatomiques et la viscosité des substances appelées à être évacuées par le drain. Il est évident que cette obstruction est gênante, car elle annule les fonctions que l'on vient d'évoquer plus haut. C'est donc une préoccupation constante des médecins d'éviter cette obstruction.

Dans le cadre particulier de la cavité abdominale, thoracique ou pelvienne, une obstruction peut se produire de trois façons différentes:

-Comme les organes intra-abdominaux, intrathoraciques ou intra-pelviens ont une certaine mobilité physiologique, ils viennent en contact du drain pour y adhérer, et ce d'autant plus que le drain est le plus souvent soumis à une aspiration pour stimuler l'évacuation des fluides;

-Les substances drainées ont une viscosité de plus en plus grande notamment en raison de la coagulation et elles adhèrent donc de plus en plus à la paroi interne du drain pour finir de la boucher;

-Du fait de son implantation, l'inclinaison du drain n'est souvent pas favorable notamment en raison de la position couchée du malade. Le drain ne bénéficie alors d'aucune déclivité et la pesanteur annule l'effet de siphonnage.

Une tentative pour fournir un drain permettant d'éviter ce fonctionnement médiocre des drains classiques est décrite dans le USA-3,114,373. Il s'agit dans ce cas d'un ensemble de drainage composé d'un tube principal fermé à son extrémité distale et raccordé à une source à dépression à son extrémité proximale. L'extrémité distale, placée dans la cavité à drainer (ici l'espace gastrointestinal) est pourvue d'une rangée d'orifices débouchant latéralement du tube et à travers lesquels sont aspirées les substances à évacuer.

Ce tube principal est flanqué d'un tube auxiliaire qui débouche dans le tube principal tout à fait à son extrémité distale fermée c'est-à-dire au delà des orifices d'évacuation. A son autre extrémité, le tube auxiliaire peut être laissé à l'air libre, connecté à une source hypodermique ou à une autre source de médication ou bien à une bouteille contenant une solution saline. L'air et/ou la solution saline peuvent ainsi s'écouler en continu à travers le tube auxiliaire dans l'espace délimité par l'extrémité distale du tube principal pour se mélanger avec les substances drainées et éviter ainsi le colmatage et la détérioration des tissus environnants, tout en maintenant dans le drian un débit d'air suffisant.

Si la solution saline peut dans une certaine mesure éviter la coagulation et dissoudre les conglomérats se formant dans ce tube d'évacuation, ces difficultés ne sont pas évitées si de l'air au lieu de cette solution est introduit dans le tube auxiliaire. Or, la nécessité d'une solution saline complique considérablement le drain lui-même et son utilisation car le personnel de surveillance doit la renouveler, la préparer ou se la procurer etc. En outre, une solution saline risque de ne pas être tolérée par les organes entourant le drain.

D'autre part, dans le cas de l'utilisation sur un site chirurgical, il est exclu d'envoyer dans le tube auxiliaire de l'air ambiant en tant que tel, car il proviendrait d'un milieu non stérile. Il faudra donc nécessairement prévoir un dispositif de stérilisation de l'air ou bien une source d'air stérile, ce qui là encore complique notablement l'installation et son utilisation.

L'invention a pour but de fournir un drain chirurgical perfectionné ne comportant pas ces inconvénients et assurant en permanence l'existence d'un passage de drainage sans que la coagulation, les résidus solides ou encore les organes environnants puissent l'obstruer.

L'invention a donc pour objet un drain chirurgical pour l'évacuation en phase postopératoire de toutes sécrétions nuisibles d'un site chirurgical, comportant un tube dont l'extrémité distale est fermée et pourvue d'orifices latéraux d'aspiration desdites sécrétions et dont l'extrémité proximale est destinée à être raccordée à une source de vide, ledit tube étant associé à une canalisation destinée à contenir un fluide chargé d'éviter l'obturation desdits orifices au cours de l'utilisation du drain, ce dernier étant caractérisé en ce que ladite canalisation est délimitée entre la paroi intérieure du tube et une gaine en une matière élastique fixée à l'intérieur dudit tube de manièreà ménager dans celui-ci un volume d'une capacité sélectivement variable et formé par au moins une poche communiquant avec une source de fluide, mais séparée du passage laissé libre dans le tube pour l'évacuation des sécrétions, ledit fluide pouvant être introduit et extrait de ladite poche pour permettre son gonflage/dégonflage.

Ainsi, grâce à l'invention, les orifices du tube de drainage peuvent être périodiquement décolmatés, soit automatiquement, par exemple par pulsation du fluide de gonflage, soit manuellement en envoyant ledit fluide dans la poche au gré des besoins constatés par le personnel de surveillance.

L'invention sera mieux comprise à la lecture de la description qui va suivre de plusieurs modes de réalisation, cette description étant faite en se référant aux dessins annexés sur lesquels:

-la figure 1 est une vue schématique d'un dispositif de drainage comprenant le drain suivant l'invention;

-les figures 1A, 1B et C sont des vues en coupe transversale du drain de la figure 1;

-la figure 2 est une vue analogue à celui de la figure 1, les poches du drain étant gonflées;

-les figures 2A, 2B et 2C sont des vues en coupe du drain de la figure 2;

-la figure 3 montre schématiquement un autre mode de réalisation du drain suivant l'invention;

-les figures 3A, 3B et 3C sont des vues en coupe du drain de la figure 3;

-la figure 4 est une vue partielle à grande échelle d'une partie du drain de la figure 3;

-la figure 5 montre un autre mode de réalisation de l'invention;

-la figure 6 illustre une variante de l'invention.

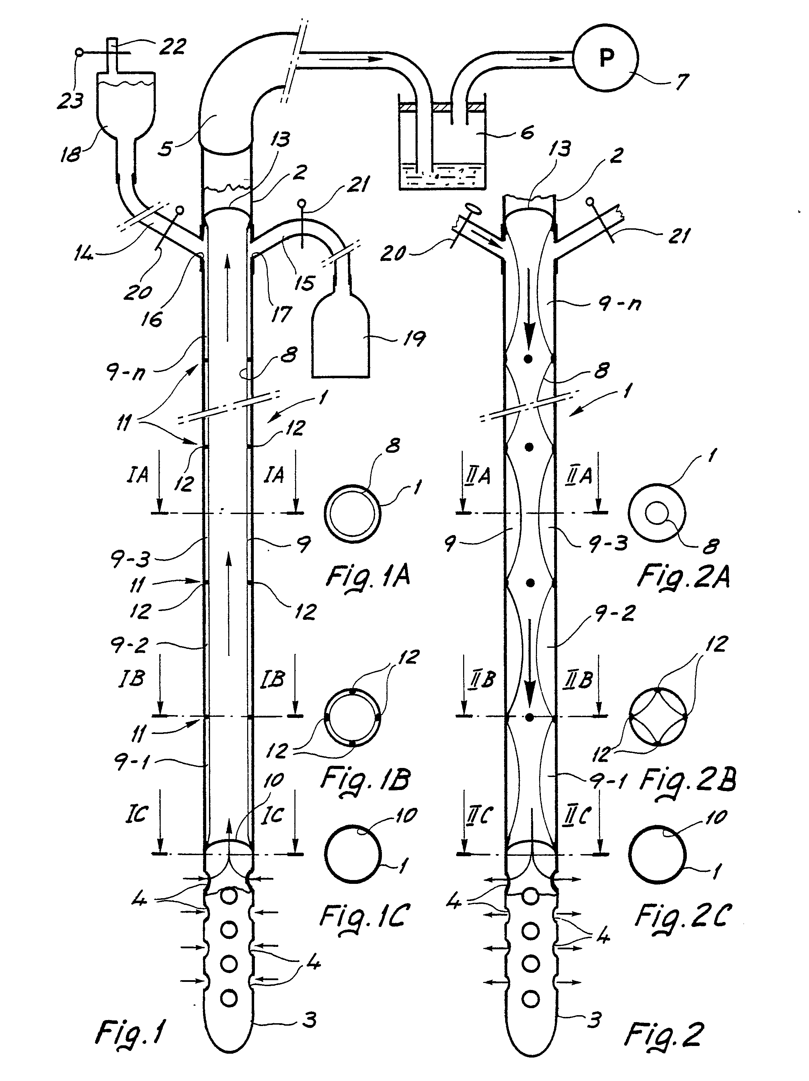

Les figures 1 à 2C représentent un premier mode de réalisation de l'invention. Dans cet exemple, le drain comporte un tube 1 ayant une extrémité proximale 2 et une extrémité distale 3. Pour fixer les idées, le tube 1 peut avoir une longueur totale de 20 à 30 cm. Il est fermé à son extrémité distale, près de laquelle sont prévus des orifices 4 faisant communiquer l'intérieur du tube 1 avec l'extérieur c'est-à-dire avec une cavité du corps humain, lorsque le tube est mis en place après une intervention chirurgicale dans la cavité en question. On peut prévoir plusieurs rangées de trous décalées l'une de l'autre de 90°, les rangées étant en quinconce selon la direction longitudinale, un nombre de 3 à 5 trous par rangée étant envisageable.

L'extrémité proximale 2 du tube est raccordée à un tuyau 5 à travers lequel les substances à évacuer de la cavité sont aspirées vers un bocal 6 qui est sous vide ou, comme représenté, raccordé à une pompe à dépression 7.

La paroi intérieure du tube 1 est doublée d'une gaine 8 en une matière élastique telle que du chlorure de polyvinyle, soudable à la matière du tube 1 qui peut être réalisé également en une matière vinyle ou encore en "Silastic" ou en caoutchouc. Cependant, le tube 1 est, de préférence, en une matière présentant une certaine rigidité.

La gaine 8 délimite dans le tube 1 un volume 9 qui est séparé de l'intérieur de celui-ci et qui s'étend sur toute la longueur du tube à l'exception d'une zone dans laquelle sont percés les orifices 4.

La gaine 8 peut être fixée à l'intérieur du tube 1 de la façon suivante. Juste au-dessus des orifices 4, elle est fixée sur tout son pourtour à la paroi du tube 1 pour former un cercle 10 de fermeture de la gaine. Entre ce cercle de fermeture 10 et l'extrémité proximale 2 du tube 1 la gaine est fixée ponctuellement à la paroi intérieure de celui-ci dans des zones de fixation 11, à l'aide de points 12. Puis, à son extrémité supérieure la gaine 8 est de nouveau fixée sur tout son pourtour à la paroi du tube 1, formant un autre cercle de fermeture 13. Ainsi, entre la paroi du tube 1 et la gaine 8 sont formées plusieurs poches 9-1, 9-2, 9-n formant ensemble le volume 9. Les cercles de fermeture 10 et 13, ainsi que les points 12 peuvent être réalisés par soudage, par collage ou par tout autre moyen de fixation approprié.

On doit noter toutefois que le volume 9 peut également être d'un seul tenant pour ne former qu'une seule poche annulaire s'étendant entre les cercles de fermeture 10 et 13. Bien entendu, dans cette variante les points de fixation 12 ne sont pas nécessaires.

Juste en dessous du cercle de fermeture 13 débouchent dans le volume 9 deux tubulures 14 et 15 à travers des orifices respectifs 16 et 17 pratiqués dans la paroi du tube 1. Dans la version représentée, les tuyaux 14 et 15 sont connectés respectivement à des bocaux 18 et 19.

Des robinets 20 et 21 sont insérés dans les tubulures 14 et 15. Sur le dessin, chacun de ces robinets est représenté symboliquement par un trait disposé en travers de la canalisation dans laquelle il est inséré, étant entendu qu'il est considéré fermé lorsque sa manette est représentée par un cercle et ouvert lorsque cette manette est symbolisée par un ovale. Il est à noter que le matériel (robinets, tubulures, bocaux, etc.) que l'on vient de décrire peut facilement être trouvé dans le commerce, car il est couramment utilisé pour les perfusions, par exemple.

Dans la configuration de la figure 1, il est supposé que le drain suivant l'invention vient d'être mis en place après une intervention chirurgicale et que les bocaux 18 et 19 sont raccordés, ainsi que le bocal 6 et la pompe à vide 7. Les robinets 14 et 15 sont dans leur position fermée, tandis que le bocal 18 est rempli d'un fluide de gonflage tel que de l'eau non stérile par exemple. Le bocal 18 possède en outre en évent 22 qui est muni d'un robinet 23 pour permettre une mise à l'air libre sélective du bocal 18.

Quelque temps après sa mise en place, le drain est susceptible de s'obstruer pour les raisons invoquées plus haut. Le personnel traitant procède alors au gonflage du volume 9 de la façon suivante. Les robinets 20 et 23 sont ouverts ce qui fait pénétrer de l'eau dans le volume 9. La gaine 8 étant souple, la pression hydrostatique la gonfle et réduit donc brusquement le volume intérieur du tube 1. Il en résulte une augmentation de pression à l'extrémité distale du drain, ce qui agit sur les tissus ayant éventuellement obturé les orifices 4 pour les en éloigner. Simultanément, les sécrétions se trouvant plus haut dans le drain sont retenues par les barrages formés successivement de bas en haut par la ou les poches et que la gaine a engendrées lorsqu'elle s'est gonflée. Ainsi, le gonflage progressif de la ou des poches formant le volume 9 provoque un mouvement de reptation de bas en haut tendant à éjecter les sécrétions et notamment les produits susceptibles de coaguler ou d'adhérer à la paroi formée par la gaine 8. On constate ainsi que le drain constitue en quelque sorte une imitation d'une veine et de ses valvules.

Pour dégonfler le volume 9, il suffit de fermer le robinet 20 et d'ouvrir le robinet 21, de manière à faire s'écouler l'eau du volume 9 dans le bocal 19. Cette opération peut être facilitée en utilisant pour ce dernier un bocal préalablement mis sous dépression.

Le personnel traitant peut répéter autant de fois que nécessaire le cycle que l'on vient de décrire, à des instants plus ou moins espacés dans le temps.

On constate qu'à aucun moment le fluide de gonflage ne peut communiquer avec le volume du drain proprement dit, le volume 9 en étant parfaitement isolé. De la sorte, le fluide de gonflage n'a pas besoin d'être stérile.

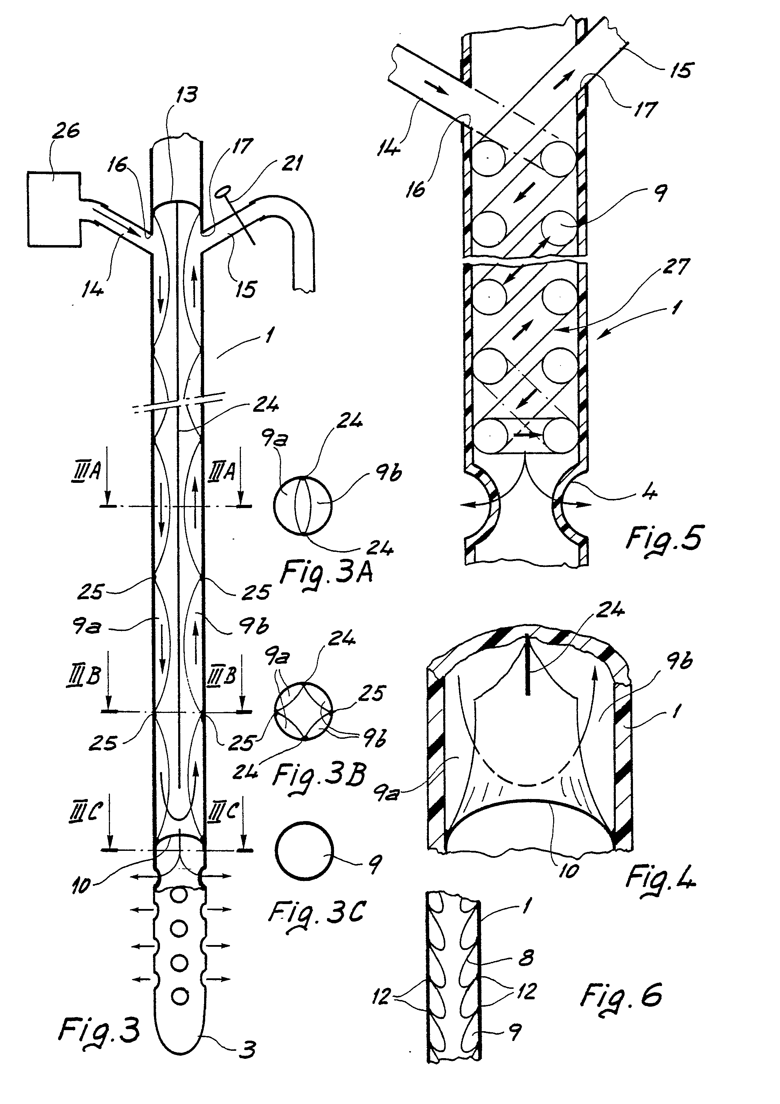

Les figures 3 à 3C et 4, représentent une variante de l'invention qui ne diffère de la réalisation des précédentes figures que par le fait que la gaine 9 est attachée sur la paroi intérieure du tube 1 par deux traits de soudure longitudinaux 24 diamétralement opposés et de distance en distance par des points de soudure 25, également diamétralement opposés mais décalés de 90° par rapport aux traits de soudure 24. En haut et en bas, la gaine 8 est fixée à la paroi du tube 1 par des cercles de fermeture 10 et 13 comme précédemment. Dans ces conditions, le volume délimité entre la paroi du tube 1 et la gaine 8 est formé par deux chambres longitudinales 9a et 9b qui ne communiquent l'une avec l'autre que près du cercle de fermeture 10.

Dans cette variante, il est supposé en outre que la tubulure 14 est raccordée à un dispositif de pompage 26 apte à engendrer une pression variable périodiquement du fluide de gonflage avec lequel le volume 9 est rempli. Il peut s'agir par exemple d'un appareil de la Marque Déposée "Vial Medical" type SE200. Cet appareil est conçu de telle manière qu'une seringue de forme classique peut y être maintenue alors que son piston peut être actionné de façon programmée par un poussoir mobile de l'appareil. Le programme d'actionnement peut être choisi au gré des besoins (fonctionnement intermittent avec réglage de la période entre deux actionnements, etc.).

L'utilisation d'un tel dispositif de pompage présente l'avantage de pouvoir rester en permanence raccordé au drain sans qu'il soit nécessaire que le personnel intervienne régulièrement. Comme déjà indiqué, la fréquence de variation de la pression pourra être réglable ainsi que la valeur moyenne de cette pression. Bien entendu, le mode de réalisation des figures 1 à 2C peut également être pourvu d'un dispositif de pompage automatique tel que le dispositif 26.

La figure 5 montre encore une autre variante de l'invention dans laquelle le volume 9 est délimité par une seule poche formée par un second tube 27 enroulé en deux hélices imbriquées communiquant l'une avec l'autre à l'extrémité distale du drain et débouchant à l'extrémité proximale, respectivement dans les tubulures 14 et 15. Ce tube 27 est également réalisé en une matière élastique de manière à pouvoir être gonflé ou dégonflé selon les besoins.

Le fluide de gonflage peut être de l'eau, de l'air, de l'oxygène, ou tout autre fluide approprié. Il n'a pas besoin d'être stérilisé. Si la pression d'aspiration imposée pour l'évacuation des sécrétions est choisie entre 15 et 20 mm Hg par exemple, une valeur de pression de gonflage du volume 9 peut être de 15 à 20 mm Hg également. Cependant des pressions d'aspiration situées entre 8 et 30 mm Hg peuvent également être envisagées.

On peut noter encore qu'un choix judicieux des endroits où on prévoit des points de fixation de la gaine 8 permet de former des sortes de ballonnets (figure 8). Ainsi, dans la configuration dégonflée, la longueur de la gaine 8 entre deux points de fixation peut être supérieure à la longueur du tube en ces deux points. Par ailleurs, la distance longitudinale entre deux points de fixation peut être plus faible que celle représentée aux figures.

## Revendications

1. Drain chirurgical pour l'évacuation en phase postopératoire de toutes sécrétions nuisibles apparaissant dans un site chirurgical, comportant un tube (1) dont l'extrémité distale est fermée et pourvue d'orifices latéraux (4) d'aspiration desdites sécrétions et dont l'extrémité proximale (2) est destinée à être raccordée à une source de vide (6, 7), ledit tube étant associé à une canalisation destinée à contenir un fluide chargé d'éviter l'obturation desdits orifices (4) au cours de l'utilisation du drain, ce dernier étant caractérisé en ce que ladite canalisation est délimitée entre la paroi intérieure du tube (1) et une gaine (8) en une matière élastique fixée à l'intérieur dudit tube (1) de manière à ménager dans celui-ci un volume (9) d'une capacité sélectivement variable et formé par au moins une poche (9-1, 9-2...9-n) communiquant avec une source de fluide, mais séparée du passage laissé libre dans le tube pour l'évacuation des sécrétions, ledit fluide pouvant être introduit et extrait de ladite poche pour permettre son gonflage/dégonflage.

2. Drain chirurgical suivant la revendication 1, caractérisé en ce que ladite gaine (8) est coaxiale au tube de manière à délimiter un volume annulaire (9) fermé contre la paroi intérieure du tube près des extrémités distale et proximale (2, 3).

3. Drain chirurgical suivant la revendication 1, caractérisé en ce que ladite gaine (8) est fixée ponctuellement et de distance en distance à ladite paroi intérieure du tube (1) de manière à créer dans ledit volume (9) une pluralité de poches communiquant les unes avec les autres (9-1, 9-2... 9-n).

4. Drain chirurgical suivant la revendication 3, caractérisé en ce que ladite gaine (8) est fixée également à la paroi intérieure du tube le long de deux génératrices diamétralement opposées à l'exception d'une zone située à sa fermeture distale (10) sur la paroi intérieure du tube (1).

5. Drain chirurgical suivant la revendication 3, caractérisé en ce qu'en configuration dégonflée, la longueur d'une portion de gaine située entre deux points de fixation adjacents (12) espacés longitudinalement est plus grande que la longueur du tube - (1) entre ces deux points (12).

6. Drain chirurgical suivant la revendication 2, caractérisé en ce que ladite gaine est fermée par deux hélices imbriquées d'un tuyau communiquant l'une avec l'autre à l'extrémité distale dudit tube et fixées sur la paroi intérieure de celui-ci.

7. Drain chirurgical suivant l'une quelconque des revendications 1 à 6, caractérisé en ce que ladite source de fluide (18; 26) communique avec ledit volume (9) à travers un orifice (16) pratiqué dans la paroi du tube près de l'extrémité proximale dudit volume.

8. Drain chirurgical suivant la revendication 7, caractérisé en ce que ledit volume (9) communique par un deuxième orifice (17) pratiqué dans la paroi du tube près de ladite extrémité proximale (2), avec un récipient (19) pour recueillir le fluide évacué dudit volume.

9. Drain chirurgical suivant l'une quelconque des revendications 1 à 8, caractérisé en ce que ladite source de fluide est pulsée.

10. Drain chirurgical suivant l'une quelconque des revendications 1 à 9, caractérisé en ce que ledit fluide est de l'air, de l'oxygène, ou de l'eau.

Fig.1A

Fig.2A

Fig.1B

Fig.2B

Fig.1C

Fig.2C

Fig.1

Fig.2

Fig.3

Fig.3A

Fig.3B

Fig.3C

Fig.5

Fig.4

Fig.6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-2 470 665 (STIEHL) <br> * Colonne 1, lignes 4-33; colonne 3, ligne 37 - colonne 4, ligne 33; figure 2 * | 1 | A 61 M 1/00 <br> A 61 M 27/00 |
| A | DE-A-1 491 755 (JACKSON) <br><br> * Page 5, ligne 8 - page 8, ligne 12; figure 1 * | 1,2,7, 10 | |
| A | US-A-3 863 641 (POPA) <br> * Colonne 1, lignes 21-24; colonne 1, lignes 56-58; figures * | 1 | |

-----

| | | | DOMAINES TECHNIQUES RECHERCHES (Int. Cl.4) |
|---|---|---|---|
| | | | A 61 M |

Le présent rapport de recherche a été établi pour toutes les revendications

| Lieu de la recherche | Date d'achèvement de la recherche | Examinateur |
|---|---|---|
| LA HAYE | 11-06-1987 | SCHOENLEBEN J.E.F. |